# EUROPEAN PATENT SPECIFICATION

(11) **EP 1 280 876 B1**
(45) Date of publication and mention of the grant of the patent: **22.03.2006**
(21) Application number: 01934997.6
(22) Date of filing: 03.05.2001
(51) Int. Cl.: C11C 5/00, A61L 9/03

(54) **INCENSE CANDLE**
WEIHRAUCHKERZE
BOUGIE D'ENCENS

(30) Priority: 03.05.2000 US 563575
(43) Date of publication of application: 05.02.2003
(73) Proprietor: S.C. JOHNSON & SON, INC., Racine, Wisconsin 53403 (US)
(72) Inventor: KANDATHIL, Thomas V., Racine, WI 53402 (US); KAWAMOTO, Ryuji, Odawar, Kanagawa 256-0802 (JP); TACZ, Maciej K., Racine, WI 53402 (US); TSURUOKA, Shuji, Georgetown, 10250 Penang (MY)
(74) Representative: Carpmaels & Ransford
(86) International application number: PCT/US2001/014184
(87) International publication number: WO 2001/083656

(56) References cited:
- US-A- 3 175 876
- US-A- 3 630 697
- US-A- 3 898 039
- US-A- 4 005 978
- US-A- 4 568 270
- US-A- 4 693 890
- US-A- 5 961 967
- US-A- 6 063 144
- DATABASE WPI Section Ch, Week 198841 Derwent Publications Ltd., London, GB; Class B07, AN 1988-290589 XP002179825 -& JP 63 214255 A (OHTSUKI M), 6 September 1988 (1988-09-06)

## Description

### BACKGROUND OF THE INVENTION

### Technical Field

The present invention relates to the dispensing of fragrances to the atmosphere from a candle product. More specifically, this invention relates to candles which diffuse incense when burning, without the formation of excessive smoke, such as is normally associated with incense.

### Background Art

Candles have been known and used since the earliest days of civilization. A typical candle is formed of a solid or semi-solid body of wax, such as paraffin wax or beeswax, and it contains an axially embedded combustible fibrous wick.

When the wick of a candle is lit, the generated heat melts the solid wax, and the resulting liquid wax flows up the wick by capillary action, and is combusted. This burning cycle becomes self-supporting, for as long as the flame is allowed to bum, and for as long as there is additional wax to fuel the flame.

In addition, wickless candles are known, in which no wick is present, and the flame feeds directly upon the wax, forming a pool of liquid wax, which is consumed by the flame. Most commonly, wickless candles are produced by extrusion, in a taper-shaped form. Wickless candles may also be extruded to form a flexible taper filament, which may be spiral wound for incorporation into a dispensing device, such as an air-freshener or insect control ingredient containing candle.

In recent years, candles have been developed to appeal to the olfactory senses as well as the visual, in that they incorporate fragrancing ingredients. This type of candle usually incorporates a fragrance oil in the wax body, which is released from the liquified wax pool at the location of combustion, by diffusion or evaporation. However, the amount of fragrance so released is not great, since the temperature of the melted wax pool surrounding a candle flame is only about 60°C, and the wax reduces the vapor pressure of fragrance oils, thereby suppressing diffusion of the fragrance. Conventional fragrance candles also have drawbacks due to cost and other considerations. The incorporation of fragrance oil in candle wax is difficult to achieve in a quantity which ensures the release of a suitable level of fragrance into the atmosphere during candle burning. Many fragrance oils include components which also soften candle waxes, resulting in an undesirable loss of rigidity in the candle structure, and limiting the concentration of fragrance oil which may be added to the candle. Aside from the softening of the paraffin wax caused by such fragrance oils, or by conventional coupling agents employed to incorporate complex perfumes into candle wax, it is known that such materials also lower the melting temperature of the wax, and thus the temperature of the pool of melted wax, which correspondingly lowers the rate of volatilization of incorporated perfumes. Further, incorporated fragrance oils tend to migrate and volatilize from the candle prematurely.

Moreover, when such candles bum, there is usually little or no odor of the fragrance oils yielded in the flame, due to the oxidation of the odor ingredients by the flame, which bums the oils, alcohols, or concentrates containing the scents, so that the odors or scents are consumed in the flame of the candle as it bums. For these reasons, the portion of the perfume in an ordinary fragrance candle which actually diffuses into the atmosphere is generally very small in comparison to the portion which is simply burned away or converted to other, odorless, compounds.

On the other hand, various incenses are known, which possess high degrees of fragrance, and have been utilized in various forms for many years to impart incense to the atmosphere. However, such incenses are conventionally burned with a considerable generation of smoke, and without the visually pleasing aspects of a candle. That is, incense usually is consumed in a smoldering manner, with the release of voluminous amounts of smoke which carry the fragrance, and without the pleasing aspects and light of a candle flame. Previous attempts to add incense to candles have failed to achieve acceptance, due to poor burning characteristics of such candles, and the large volumes of smoke generated at the flame of such candles. One factor in this failure is the fact that incense compositions are poorly soluble in common candle waxes, such as paraffin, resulting in non-homogenous mixtures. These mixtures show poor burning properties and are inferior in terms of creating a desired ambiance.

Fredericks, in U. S. Patent No. 3,175,876, teaches the inclusion of carbonaceous carriers (i.e. sugars) for scents, which carriers gravitate toward the wick and release scent on the burning and smoldering of the wick. The patent also teaches the previously unsuccessful attempts at inclusion of incense in the candle to be released upon extinction and smoldering of the wicking, but teaches that scents may be carried to the flame, or to a smoldering wick, by incorporation of an appropriate carrier.

Lin, in U. S. Patent No. 3,898,039, teaches a fumigant-bearing substrate which in combination with a candle is particularly useful as a fragrance candle, wherein the fumigant is defined as inclusive of fragrances such as perfumes or incense, which may be in the form of a microencapsulated solid, liquid, or gas adhered to the substrate surface. The fumigant is released by diffusion upon exposure of the substrate to the heat of the lighted candle. In this case, the substrate bearing the fumigant is preferably substantially parallel to the longitudinal axis of the candle, and is heated by the flame, by both conduction and by radiation, to cause release of the fumigant.

In U. S. Patent No. 4,427,366, Moore teaches a scented candle comprising a bowl containing a centered candle surrounded by odorizing chips which release scent in response to the heat of the burning of the candle. Similarly, Ansari et al, in U. S. Patent No. 5,891,400, teach a volatile substance dispenser including an inner open-top container for holding a candle, and a surrounding container of a gel containing a fragrance material to be released as a result of thermal energy from the candle.

Thus, there is a continuing interest in the development of improved fragrance candles, and a need exists for acceptable incense burning candles.

### BRIEF SUMMARY OF THE INVENTION

The invention provides an incense burning candle, in which the incense is incorporated directly into the candle wax, for release upon combustion and formation of a liquid pool of wax. That is, the incense incorporated in the candle is released to the atmosphere by volatilization from the liquid pool of wax formed upon burning of the candle, in the same manner that other fragrances are released from fragrance containing candles. The present invention overcomes the difficulty of the low solubility of incense in candle wax by the addition of special components, or coupling agents. Further, the present invention overcomes the problem of the low release rate of the incense, which is the result of low pool temperature, by the proper selection and concentration of incense and coupling agent.

Accordingly, the invention provides an incense candle comprising:
(a) from about 75 to about 97 weight percent of a candle wax;
(b) from about 1 to about 12 weight percent of an incense ingredient selected from the group consisting of myrrh, cedarwood, cedrenol, cedrol, birch, methyl salicylate, fir balsam, sandalwood, santalol, juniper, benzoin, coniferyl benzoate, thyme, thymol, bay, eugenol, myrcene, basil, camphor, methyl cinnamate, cinnamon, cinnamic aldehyde, rosemary, clove, borneol, and mixtures thereof;
(c) from about 1.0 to about 5 weight percent of a coupling agent wherein said coupling agent is selected from the group consisting of stearic acid, stearyl alcohol, polyethylene-polyvinyl acetate copolymers, fatty acid esters, soy bean oil waxes, and mixtures thereof; and
(d) up to about 10 weight percent of additives;
wherein the incense ingredient is incorporated directly into the candle wax, and is released into the atmosphere from the wax pool formed upon combustion of said candle to thereby create a perception of burning incense.

Thus, an object of the present invention is to provide a source of incense which may be released to the atmosphere without the normally associated smoldering and smoke of commonly used incense powder, sticks, cones, or coils. It is also an object of the present invention to provide a source of intense fragrancing which provides illumination as well as fragrance, with little or no objectionable smoke or soot. It is further an object of this invention to provide a candle, in the form of a jar candle or free standing as a votive, column, or taper candle, which bums smoothly with a steady and consistent flame and little or no smoke generation, and releases a strong incense fragrance.

These and still other objects and advantages of the present invention will be apparent from the description which follows. The following description is merely of the preferred embodiments. Thus, the claims should be looked to in order to understand the full scope of the invention.

### DETAILED DESCRIPTION OF THE INVENTION

One or more objects of the present invention are accomplished by the provision of an incense candle comprising:
(a) from about 75 to about 97 weight percent of a candle wax;
(b) from about 1 to about 12 weight percent of an incense ingredient selected from the group consisting of myrrh, cedarwood, cedrenol, cedrol, birch, methyl salicylate, fir balsam, sandalwood, santalol, juniper, benzoin, coniferyl benzoate, thyme, thymol, bay, eugenol, myrcene, basil, camphor, methyl cinnamate, cinnamon, cinnamic aldehyde, rosemary, clove, borneol, and mixtures thereof;
(c) from about 1 to about 5 weight percent of a coupling agent wherein said coupling agent is selected from the group consisting of stearic acid, stearyl alcohol, polyethylene-polyvinyl acetate copolymers, fatty acid esters, soy bean oil waxes, and mixtures thereof; and
(d) up to about 10 weight percent of additives;
wherein the incense ingredient is incorporated directly into the candle wax, and is released into the atmosphere from the wax pool formed upon combustion of said candle to thereby create a perception of burning incense.

The candle product may take the form of a taper candle, a votive candle, or a column candle, or may be contained in a jar or other container. Such candles preferably contain a wick, but could also comprise a wickless taper. The wick, if present, may be any conventional consumable wick material, such as cotton, cellulose, nylon, or paper. The wick may preferably be located in the center of the candle, or may be off-center as desired. The presence of two or more wicks is also within the scope of the present invention. Nonconsumable wicks of an appropriate design, such as fiberglass, could conceivably be used, but in the past have been found to present problems of poor burn characteristics and excessively large flame when employed in conventional candles.

The candle wax ingredient may be selected from commercially available wax media. The combustible body of a candle product typically is a thermoplastic blend of organic materials such as tallow, beeswax, paraffin wax, montan wax, carnauba wax, and microcrystalline wax or mixtures thereof. The wax is preferably a paraffin wax or other less costly wax commonly employed for use in candles, such as tallow or beeswax, and is present in an amount of from about 75 to 97 weight percent, preferably from about 80 to 95 percent, and most preferably from about 90 to 92 percent. A preferred candle wax of the present invention is a blend of a hydrocarbon wax and a microcrystalline wax, such as IGI Parafflex 4639, a product of The international Group, Inc., believed to comprise a blend of macro- and micro- crystalline paraffin waxes. Other paraffin waxes, like Exxon SRW 128 and similar waxes, can be used in the product according to the present invention provided that an appropriate coupling agent is added, as set forth herein.

The use of a coupling agent, or solubilizer, which is soluble in conventional candle waxes and in which incense ingredients are soluble, has been found to be critical to the incorporation of incense into the candle wax. Absent such a coupling agent, incense fragrances exhibit poor solubility in conventional hydrocarbon waxes, resulting in a poor burning composition which is overly soft for candle formation. Suitable coupling agents for use with incense ingredients are selected from the group consisting of stearic acid, stearyl alcohol, polyethylene-polyvinyl acetate copolymers, fatty acid esters, and soy bean oil derivatives. Known coupling agents which have been used in the past, such as di-isopropyl adipate, isopropyl palmitate, d-limonene, linear alcohols having from 8 to 12 carbon atoms, diethyl phthalate, benzyl benzoate, and microcrystalline waxes, are unsuitable for use with the paraffin type waxes preferably employed in candles, due to issues of poor flame characteristics and softening of the wax, among others. In addition, the coupling agents used in the present invention have been found to minimize syneresis, i.e. the separation of the fragrance ingredient from the solidified wax in the form of a liquid. In the past, when melted waxes having fragrance ingredients blended therein were allowed to cool, the fragrance oils would separate out from the wax, often forming an unsightly oily surface layer, but also exposing the fragrance oil to the flame per se.

A preferred coupling agent is available under the trade name ELVAX, a product of DuPont, believed to be a copolymer of vinyl acetate and polyethylene. Such a coupling agent is necessary to achieve solubility of the incense in the candle wax, but also functions to add oxygen to the composition, improving the bum characteristics and reducing smoking. It is believed that the presence of a coupling agent increases the solubility of high-molecular weight resins present in the fragrance compositions, without compromising the hardness of candle wax. Additives having increased polarity, and at least partial compatibility with the paraffin wax, like fatty alcohols or fatty acid esters, are also suitable as coupling agents. Natural waxes, based on both fatty acid esters of fatty alcohols and saturated fats, can also be used as coupling agents in the present invention. No paraffin wax, however, can be used as the principle candle wax ingredient in the product without an appropriate coupling agent. The coupling agent is present in a concentration of from 1 to 5 weight percent, preferably 4 percent, and most preferably from 1 to 2 percent.

The incense ingredient comprises the stated fragrance ingredients, or mixtures thereof, which may be used to create a sensory perception of incense. The term incense ingredient shall be understood to encompass fragrance ingredients and compositions inclusive thereof. The incense ingredients that are essential to achieve such an incense perception are selected from, myrrh, cedarwood, cedrenol, cedrol, birch, methyl salicylate, fir balsam, sandalwood, santalol, juniper, benzoin, coniferyl benzoate, thyme, thymol, bay, eugenol, myrcene, basil, camphor, methyl cinnamate, cinnamon, cinnamic aldehyde, rosemary, clove, and borneol, Such fragrance materials are frequently referred to as natural resins and are characterized by relatively high molecular weights, and the presence of aromatic, condensed ring structures incorporating the presence of polar groups, such as alcohols, carbonyls, aldehydes, ketones, esters, etc., which are incompatible with paraffin waxes, but are highly soluble in the coupling agents employed in the present invention. Such incense ingredients are present in concentrations of from about 1 to about 12, preferably from about 3 to about 10 weight percent of the composition of the candle, more preferably from about 5 to about 9 percent, and most preferably from about 6 to about 8 percent. The preferred incense ingredients are selected from the group consisting of myrrh, cedarwood, methyl salicylate, fir balsam, sandalwood, juniper, coniferyl benzoate, bay, eugenol, cinnamon, cinnamic aldehyde, rosemary, and clove, and mixtures thereof. Because of the lower volatility of incense-type fragrances, as compared to other more common types of fragrances, the concentration of the incense ingredient in the candle composition is preferably above about 5 weight percent.

The term "incense" has a double meaning in common usage, which may indicate either a characteristic type of resinous, woody, herbaceous odor, produced during the burning and/or smoldering of solid, natural materials (mostly different kinds of wood and herbs, traditionally in the form of a stick, cone, coil or powder) or these materials themselves; hence the terms "buming incense" or "smelling incense". Incense was traditionally used in ancient cultures (especially in Eastern Asia) for religious practices; but it has now become popular all over the world as a relaxation aid or form of aroma therapy. The novelty of the present invention is that never before has incense been associated with candle burning. These two activities were always separate, due to the incompatibility of incense materials with paraffin wax. In the present invention, the applicants have essentially "extracted" the incense odor obtained by actually burning incense, and successfully incorporated it into a fragranced candle. Incense candles in accordance with the present invention constitute a different form of odor-diffusing device, of a nature much more widely accepted and used among the various cultures of the world than are conventional incense burning devices.

In addition to the candle wax, incense, and coupling agent, other additives which are commonly used in candles may be added, up to about 10 percent by weight of the candle composition. Such additives may include polymeric additives such as thermoplastic resins, adapted for fiber-formation by processes such as extrusion or compression molding, and coloring agents. Other additive ingredients may include UV absorbers (such as UVINUL®, available from BASF, or CYASORB®, available from Cytec Industries, which are believed to be benzophenone derivatives), and antioxidants, which may be present to protect any dye or fragrance from degradation. Preferred ranges of additive are from about 0 to about 7 percent, and most preferably from about 0 to about 1 percent by weight.

### EXAMPLES

Candles were prepared, using the compositions as set forth in Table 1, in weight percentages. The process for preparation of the candles of the present invention includes melting the wax, blending in the additives, induding the coupling agent, mixing in the incense ingredient(s), and pouring in the form of jar candles. A consumable wick was employed in each of the following examples, placed in each candle before the wax had hardened. Where the copolymer coupling agent (a copolymer of polyethylene and polyvinyl acetate) was employed, the wax was required to be heated to a temperature of from about 150-165° F. for the additive to dissolve. In comparative Example 5, a soybean oil wax was utilized as a coupling fuel, in which the incense fragrance was highly soluble. However, soybean oil derived waxes are not considered particularly suitable for use as a candle wax, due to relatively high cost, objectionable coloration, and tendency toward cracking upon cooling and solidification.

Candles prepared in accordance with the Examples were burned, and provided a satisfactory perception of incense burning. These candles were readily lit, and burned normally, with the appearance of conventional candles, but with the perception of a strong sandlewood odor released. Such incense odors have not been previously available in the form of a candle, and previously have only been available from conventional incense burning.

**TABLE 1**

| | 1 | 2 | 3 | 4 | 5 |
|---|---|---|---|---|---|
| **Candle wax** | | | | | |
| IGI 4639 | 91.75 | | | | |
| Astor Wax | | 90.75 | | | |
| Paraffin wax SRW 128 | | | 90.75 | 90.75 | |
| Soybean oil Wax | | | | | 92.75 |

| **Fragrance** | | | | | |
|---|---|---|---|---|---|
| Firmenich Kamogawa | 7.00 | | 7.00 | 7.00 | |
| Firmenich Kyoto | | 7.00 | | | 7.00 |

| **Coupling agent** | | | | | |
|---|---|---|---|---|---|
| Copolymer | 1.00 | 2.00 | 1.00 | | |
| Stearic acid | | | 1.00 | | |
| Stearyl alcohol | | | | 2.00 | |

| **Additive** | | | | | |
|---|---|---|---|---|---|
| Uvinul® 3008 | 0.25 | 0.25 | 0.25 | 0.25 | 0.25 |

### INDUSTRIAL APPLICABILITY

The incense candles of the present invention are useful in the home to create a pleasing environment and to provide both light and fragrance to the atmosphere. Such candles may be produced using conventional candle making methods and equipment, and utilize readily available materials in their production.

## Claims

1. An incense candle comprising:
(a) from 75 to 97 weight percent of a candle wax;
(b) from 1 to 12 weight percent of an incense ingredient selected from the group consisting of myrrh, cedarwood, cedrenol, cedrol, birch, methyl salicylate, fir balsam, sandalwood, santalol, juniper, benzoin, coniferyl benzoate, thyme, thymol, bay, eugenol, myrcene, basil, camphor, methyl cinnamate, cinnamon, cinnamic aldehyde, rosemary, clove, borneol, and mixtures thereof;
(c) from 1 to 5 weight percent of a coupling agent wherein said coupling agent is selected from the group consisting of stearic acid, stearyl alcohol, polyethylene-polyvinyl acetate copolymers, fatty acid esters, soy bean oil waxes, and mixtures thereof; and
(d) up to 10 weight percent of additives;
wherein the incense ingredient is incorporated directly into the candle wax, and is released into the atmosphere from the wax pool formed upon combustion of said candle to thereby create a perception of burning incense.

2. The incense candle of claim 1, wherein said additives are selected from the group consisting of coloring agents, UV absorbers, antioxidants, fiber forming polymeric additives and mixtures thereof.

3. The incense candle of claim 1 or claim 2, comprising from 5 to 9 weight percent incense ingredient.

4. The incense candle of any preceding claim, wherein said incense ingredient is selected from the group consisting of myrrh, cedarwood, methyl salicylate, fir balsam, sandalwood, juniper, coniferyl benzoate, bay, eugenol, cinnamon, cinnamic aldehyde, rosemary, and clove, and mixtures thereof.

5. The incense candle of any preceding claim comprising from 80 to 95 weight percent of a candle wax selected from the group consisting of tallow, beeswax, paraffin wax, and mixtures thereof.

6. The incense candle of any preceding claim, comprising from 1 to 2 percent of said coupling agent.

7. The incense candle of any preceding claim, comprising from 6 to 8 percent of said incense ingredient.

8. The incense candle of any preceding claim, configured as a container candle.

## Patentansprüche

1. Weihrauchkerze, umfassend:
(a) 75 bis 97 Gewichtsprozent eines Kerzenwachses;
(b) 1 bis 12 Gewichtsprozent eines Weihrauchbestandteils, der aus der Gruppe ausgewählt ist, die aus Myrrhe, Zedernholz, Cedrenol, Cedrol, Birke, Methylsalicylat, Tannenbalsam, Sandelholz, Santalol, Wacholder, Benzoin, Coniferylbenzoat, Thymian, Thymol, Lorbeer, Eugenol, Myrcen, Basilikum, Campher, Methylcinnamat, Zimt, Zimtaldehyd, Rosmarin, Nelke, Borneol und deren Gemischen besteht;
(c) 1 bis 5 Gewichtsprozent eines Haftmittels, wobei das Haftmittel aus der Gruppe ausgewählt ist, die aus Stearinsäure, Stearylalkohol, Polyethylen-Polyvinylacetat-Copolymeren, Fettsäureestern, Sojabohnenölwachsen und deren Gemischen besteht;
(d) bis zu 10 Gewichtsprozent Additive;
wobei der Weihrauchbestandteil direkt in das Kerzenwachs eingebracht und aus dem Wachspool beim Verbrennen der Kerze in die Atmosphäre freigesetzt wird, wodurch eine Empfindung von brennendem Weihrauch erzeugt wird.

2. Weihrauchkerze nach Anspruch 1, wobei die Additive aus der Gruppe ausgewählt sind, die aus Färbemitteln, UV-Absorptionsmitteln, Antioxidantien, faserbildenden, polymeren Additiven und deren Gemischen besteht.

3. Weihrauchkerze nach Anspruch 1 oder 2, die aus 5 bis 9 Gewichtsprozent Weihrauchbestandteil besteht.

4. Weihrauchkerze nach einem der vorhergehenden Ansprüche, worin der Weihrauchbestandteil aus der Gruppe ausgewählt wird, die aus Myrrhe, Zedernholz, Methylsalicylat, Tannenbalsam, Sandelholz, Wacholder, Coniferylbenzoat, Lorbeer, Eugenol, Zimt, Zimtaldehyd, Rosmarin und Nelke besteht.

5. Weihrauchkerze nach einem der vorhergehenden Ansprüche, die 80 bis 95 Gewichtsprozent eines Kerzenwachses umfasst, das aus der Gruppe ausgewählt ist, die aus Talg, Bienenwachs, Paraffinwachs und deren Gemischen besteht.

6. Weihrauchkerze nach einem der vorhergehenden Ansprüche, die 1 bis 2 Prozent Haftmittel umfasst.

7. Weihrauchkerze nach einem der vorhergehenden Ansprüche, die 6 bis 8 Prozent Weihrauchbestandteil umfasst.

8. Weihrauchkerze nach einem der vorhergehenden Ansprüche, die als Behälterkerze konfiguriert ist.

## Revendications

1. Bougie d'encens comprenant :
(a) de 75 à 97 % en poids d'une cire à bougie ;
(b) de 1 à 12 % en poids d'un ingrédient de type encens choisi dans le groupe constitué par la myrrhe, le cèdre, le cédrénol, le cédrol, le bouleau, le salicylate de méthyle, le baume de sapin, le bois de santal, le santalol, le genévrier, le benjoin, le benzoate de coniféryle, le thym, le thymol, le laurier, l'eugénol, le myrcène, le basilic, le camphre, le cinnamate de méthyle, la cannelle, l'aldéhyde cinnamique, le romarin, le giroflier, le boméol, et leurs mélanges ;
(c) de 1 à 5 % en poids d'un agent de couplage, lequel agent de couplage est choisi dans le groupe constitué par l'acide stéarique, l'alcool stéarylique, les copolymères polyéthylène-acétate de polyvinyle, les esters d'acides gras, les cires d'huile de soja, et leurs mélanges ; et
(d) jusqu'à 10 % en poids d'additifs ;
dans laquelle l'ingrédient de type encens est directement incorporé dans la cire à bougie, et est libéré dans l'atmosphère à partir de la cire fondue formée après combustion de ladite bougie pour ainsi créer une perception d'encens en train de brûler.

2. Bougie d'encens selon la revendication 1, dans laquelle lesdits additifs sont choisis dans le groupe constitué par les agents colorants, les absorbeurs d'UV, les antioxydants, les additifs polymères fibrogènes et leurs mélanges.

3. Bougie d'encens selon la revendication 1 ou la revendication 2, comprenant de 5 à 9 % en poids d'ingrédient de type encens.

4. Bougie d'encens selon l'une quelconque des revendications précédentes, dans laquelle ledit ingrédient de type encens est choisi dans le groupe constitué par la myrrhe, le cèdre, le salicylate de méthyle, le baume de sapin, le bois de santal, le genévrier, le benzoate de coniféryle, le laurier, l'eugénol, la cannelle, l'aldéhyde cinnamique, le romarin et le giroflier, et leurs mélanges.

5. Bougie d'encens selon l'une quelconque des revendications précédentes, comprenant de 80 à 95 % en poids d'une cire à bougie choisie dans le groupe constitué par le suif, la cire d'abeille, la cire de paraffine, et leurs mélanges.

6. Bougie d'encens selon l'une quelconque des revendications précédentes, comprenant de 1 à 2 % dudit agent de couplage.

7. Bougie d'encens selon l'une quelconque des revendications précédentes, comprenant de 6 à 8 % dudit ingrédient de type encens.

8. Bougie d'encens selon l'une quelconque des revendications précédentes, configurée sous forme de bougie contenue dans un récipient.
